# EUROPÄISCHE PATENTSCHRIFT

(11) **EP 1 177 106 B1**
(45) Veröffentlichungstag und Bekanntmachung des Hinweises auf die Patenterteilung: **29.11.2006**
(21) Anmeldenummer: 00926846.7
(22) Anmeldetag: 08.04.2000
(51) Int. Cl.: B41M 5/24, B41M 5/26

(54) **VERFAHREN ZUM BESCHRIFTEN EINES FLÄCHIGEN KLEBENDEN TRANSDERMALEN THERAPEUTISCHEN SYSTEMS AUS EINEM POLYMER**
METHOD FOR LABELING A TWO-DIMENSIONAL TRANSDERMAL THERAPEUTIC POLYMER ADHESIVE SYSTEM
PROCEDE D'INSCRIPTION SUR UN SYSTEME ADHESIF, PLAN THERAPEUTIQUE TRANSDERMIQUE ET A BASE DE POLYMERE

(30) Priorität: 23.04.1999 DE 19918473
(43) Veröffentlichungstag der Anmeldung: 06.02.2002
(73) Patentinhaber: LTS LOHMANN Therapie-Systeme AG, 56626 Andernach (DE)
(72) Erfinder: BECHER, Frank, D-56072 Koblenz (DE)
(74) Vertreter: Flaccus, Rolf-Dieter
(86) Internationale Anmeldenummer: PCT/EP2000/003152
(87) Internationale Veröffentlichungsnummer: WO 2000/064684

(56) Entgegenhaltungen:
- EP-A- 0 688 678
- EP-A- 0 987 121
- WO-A-97/44196
- DE-A- 19 630 478
- "RECHNERGESTEUERTE LASERBESCHRIFTUNGSGERATE" KUNSTSTOFFE,DE,CARL HANSER VERLAG. MUNCHEN, Bd. 78, Nr. 9, 1. September 1988 (1988-09-01), Seite 771 XP000001238 ISSN: 0023-5563

## Beschreibung

Verfahren zum Beschriften eines flächigen klebenden Systems aus einem Polymer, nämlich eines transdermalen therapeutischen Systems

Die Erfindung betrifft ein Verfahren zum Beschriften eines flächigen klebenden Systems aus einem Polymer, bevorzugt auf der der klebenden Seite abgewandten Seite, mit Informationen, z.B. Angaben zur Identifizierung des Systems als solchem oder des zu beklebenden Objekts.

Es gibt viele verschiedene Arten von flächigen klebenden Systemen aus einem Polymer, beispielsweise
- Klebefolien, z.B. (unter Umständen transparente) Klebefolien zur Kennzeichnung von Switchboards und technischen Geräten,
- technische Klebestreifen bzw. -bänder,
- Fixierpflaster (z.B. zum Fixieren von Verbänden) zur Anwendung auf der Haut,
- Wundpflaster mit Wundauflage,
- Pflaster mit Wirkstoffen wie Rheumapflaster zur topischen Aufbringung von Wirkstoffen auf die Haut,
- Pflaster mit Wirkstoffen zur systemischen Applikation von Wirkstoffen auf und durch die Haut, sogenannte transdermale therapeutische Systeme (TTS),
- Pflanzenschutzpflaster mit Wirkstoffen zur Applikation von Pflanzenschutzmitteln.

Bei flächigen klebenden Systemen besteht ein Bedürfnis, Informationen aufzubringen, z.B. über ihre Eigenschaften, ihre Anwendung, ihre Unverträglichkeiten, Lagerbedingungen und Verfallszeit, sowie gegebenenfalls Besonderheiten des zu beklebenden Objekts (Etikettenfunktion), bevorzugt auf der der klebenden Seite abgewandten Seite.

Auch bei flächigen klebenden Systemen wie transdermalen therapeutischen Systemen mit Wirkstoffen besteht ein Bedürfnis und fallweise ein zwingendes Erfordernis, derartige Pflaster nach der Anwendung, der Wirkstoffart, dem Wirkungs- und Nebenwirkungsspektrum, dem Unverträglichkeitspotential, dem Allergiepotential, den Lagerbedingungen, der Verfallszeit sowie Informationen über den Patienten usw. identifizierbar zu beschriften und damit zu kennzeichnen.

Im folgenden wird der Ausdruck "Beschriften" als Synonym für jede Art von Kennzeichnung, also auch Markierungen, Symbole, Barcodes usw., verstanden.

Beim Stand der Technik ist es üblich, solche klebenden Systeme mit beispielsweise einer Drucktechnik wie Siebdruck oder Tampondruck zu bedrucken und zu kennzeichnen. Die bekannte Technik hat jedoch eine Reihe von Nachteilen:
- die Druckfarbe braucht Zeit zum Trocknen,
- Druckfarbe haftet nur unzureichend dauerhaft auf dem hierfür vorgesehenen Polymer-Substrat,
- bei Änderung der Beschriftung entstehen lange Umrüstzeiten,
- der Aufpreßdruck beim Druckvorgang kann einen negativen Effekt auf das flächige klebende System, die enthaltenen Inhaltsstoffe wie Kleber, Zusatzstoffe wie Weichmacher und Enhancer oder den Wirkstoff haben,
- Farbzeichen auf der Polymerunterlage werden leicht verwischt, insbesondere bei Berührungen mit anderen Stoffen,
- die meisten anwendbaren Drucktechniken können nur getaktet erfolgen, wodurch die Geschwindigkeit der Produktion verringert wird,
- die Bedruckung der flächigen klebenden Systeme kann wegen der geringen Arbeitsbreite der angewandten Drucktechniken nicht auf den breiten Bahnen der Klebefolien, sondern erst nach Vereinzelung erfolgen.

Bekannt sind weiterhin Tintenstrahldrucker, für die zum Teil die gleichen, aber auch andere Nachteile gelten:
- die Druckfarbe braucht Zeit zum Trocknen,
- die Druckfarbe haftet nur unzureichend dauerhaft auf dem hierfür vorgesehenen Polymer-Substrat,
- Farbzeichen auf der Polymerunterlage werden leicht verwischt, insbesondere bei Berührungen mit anderen Stoffen,
- die Arbeitsgeschwindigkeit ist gering.

Bekannt ist auch eine Beschriftungstechnik mittels beweglich geführtem Laserstrahl mit Tonern, bei denen der Toner thermisch, z.B. auf Papier, fixiert wird; Toner haftet jedoch nur unzureichend auf den meisten Polymerunterlagen. Bekannt ist fernerhin eine Beschriftungstechnik mittels beweglich geführtem Laserstrahl, beispielsweise auf Metalluntergrund, insbesondere auf Leichtmetalluntergrund. Dabei entwickelt der Laserstrahl mittels punktuell extrem intensiver Lichtemission so hohe Temperaturen, daß das Kristallgefüge des Metalls oberflächig so verändert wird, daß das Metall eine andere Farbe annimmt.
Laserstrahlgeräte werden bisher auch beispielsweise zum Aufbringen von Buchstaben oder Zeichen auf mit einer verhältnismäßig dicken Kunststoff- oder Gummischicht umkleideten Elektrokabeln aufgebracht. Bekannt sind Lasergeräte zur Markierung von Kabeln mit Informationen im Wege einer Inline-Laser-Markierung, die mit einem frei programmierbaren Matrixsystem arbeiten, was die Wiedergabe nahezu beliebiger Schriftzeichen und Symbole gestattet. Dabei sind Geschwindigkeiten von bis zu 450 m/min üblich.

EP-A-0 688 678 offenbart ein Einschichtlaseretikett aus einer Trägerschicht aus Kunststoff, die ein Addition enthält, das unter Laserbestrahlung einem Farbumschlag zeigt. Die Trägerschicht ist einseitig mit einer Selbstklebemasse beschichtet. Für die Erzeugung des Farbumschlags ist ein geringerer Energiebedarf erforderlich, verglichen mit Lasergravurverfahren.

Wegen möglicher schädlicher Einwirkungen des Laserstrahls auf einer Schriftunterlage aus einem dünnen, mit Inhaltsstoffen wie Kleber, Weichmacher, Enhancer und Wirkstoffen versehenen Polymer und der Eindringtiefe des Strahls wurde bisher von einer tonerfreien Laserbeschriftung auf dünne flächige klebende Systeme aus Polymeren kein Gebrauch gemacht. Bei allen Klebern wird befürchtet, daß es unter Hochtemperatureinfluß zu Reaktionen der Klebermasse mit Restmonomeren kommt, bei Heißscbmelzklebern wird befürchtet, daß ihre Klebkraft durch Hitzeeinwirkung verschlechtert wird.

Hiervon ausgehend ist es Aufgabe der Erfindung, bei einem Verfahren der im Oberbegriff von Anspruch 1 genannten Art für die Beschriftung eines klebenden Systems aus Polymeren mit Hilfe eines beweglich geführten Laserstrahls Arbeitsparameter bzw. Durchführungsbedingungen anzugeben, die geeignet sind, eine schädliche Veränderung des flächigen klebenden Systems, beispielsweise durch eine die Impermeabilität der Rückschicht aufhebende Perforierung, negative Veränderungen von Rückschicht und Inhaltsstoffen wie Kleber bzw. eine thermische Veränderung der Zusatzstoffe wie Weichmacher, Enhancer oder des Wirkstoffs, sicher zu vermeiden.

Zur Lösung der Aufgabe wird bei einem Verfahren der im Oberbegriff von Anspruch 1 genannten Art mit der Erfindung vorgeschlagen, daß das Beschriften eines transdermalen therapeutischen Systems mit Hilfe eines beweglich geführten Laserstrahls in der Weise vorgenommen wird, daß eine schädliche Beeinflussung der im transdermalen therapeutischen System enthaltenen Wirkstoffe durch vom Laserstrahl erzeugte Wärme vermieden und zu diesem Zweck dessen Intensität und Eindringtiefe nach Maßgabe der Materialbeschaffenheit des transdermalen therapeutischen Systems so eingestellt wird, daß der Laserstrahl nicht bis zu der wirkstoffhaltigen Schicht des TTS durchdringt. Bei den Wirkstoffen handelt es sich um Wirkstoffe zur systemischen Applikation auf und durch die Haut.

Die Beschriftung kann auch durch Negativzeichen erfolgen, das heißt durch Herausarbeiten von Buchstaben und Zeichen aus Flächen, die nicht vom Laserstrahl beeinflußt werden. Mit Vorteil ist weiter vorgesehen, daß die Beschriftung unmittelbar nach Fertigung der breiten Rückschichtfolienbahnen oder in einem beliebigen späteren Fertigungsschritt - nach dem Beschichten, Zukaschieren anderer Folien, Schneiden in Schmalrollen oder nach dem Vereinzeln als letztem Arbeitsschritt - vorgenommen wird.
Dabei können durch Anordnung mehrerer Beschriftungseinheiten nebeneinander, versetzt, oder gestaffelt beliebige Arbeitsbreiten, also auch breite Bahnen von Klebefolien, abgedeckt werden.

Eine vorteilhafte Ausgestaltung des Verfahrens sieht vor, daß die zu beschriftende Materialschicht mit einer Überlagerungsschicht, z.B. mit einer aufgedruckten Farbschicht, überdeckt wird, die so ausgewählt ist, daß sie schon bei vergleichsweise moderater Laserbestrahlung zerfällt und dabei auf dem Hintergrund der darunterliegenden Materialschicht die gelaserten Schriftzeichen erkennen läßt.

Hierdurch ist eine besonders kontrastreiche und das Erkennen von Schriftzeichen erleichternde Schrift mit einem Minimum an Laserenergie herstellbar.
Dabei kann der optische Effekt dadurch noch verbessert werden, daß die Überlagerungsschicht gegenüber der zu beschriftenden Materialschicht mit einer augenfälligen Färbung versehen wird.

Eine weitere vorteilhafte Ausgestaltung des Verfahrens ist dadurch gekennzeichnet, daß eine Abstimmung der die Wirkungsintensität des Laserstrahls auf der zu beschriftenden Materialschicht bestimmenden Parameter wie Strahlungsenergie und geschwindigkeitsabhängige Einwirkungsdauer des Laserstrahls so vorgenommen wird, daß nur die obersten Materialschichten verändert und ansonsten keine Veränderungen der weiteren Untergrundschichten verursacht werden. Insbesondere wird mit dieser Maßnahme eine unzulässige Perforation der Rückschicht des flächigen klebenden Systems/Pflasters verhindert und eine schädliche Beeinflussung von Inhaltsstoffen wie Kleber, Zusatzstoffen und gegebenenfalls Wirkstoffen vermieden.

Eine weitere erfindungswesentliche Ausgestaltung des Verfahrens nach der Erfindung sieht vor, daß der Laserstrahl mittels elektromagnetischer Steuerung so geführt wird, daß zu jeder Zeit einzelne Zeichen oder Schriftzüge nach Programm einer zentralen Steuereinheit eingegeben oder geändert, und insbesondere Schriftzeichen von Hand über eine EDV-gesteuerte schreibmaschinenähnliche Tastatur ("Keybord") eingegeben werden können.
Die Möglichkeit, über manuelle Eingabe Schriftzeichen und Datensätze in ein zu beschriftendes flächiges klebendes System/Pflaster wie mit einer Schreibmaschine eingeben zu können, wird mit der Erfindung erstmals möglich und ist von besonderem Vorteil.

Schließlich wird mit dem Verfahren nach der Erfindung vorgesehen, daß zur Erzeugung eines ein- oder mehrfarbigen Schrift- oder Zeichenmusters wenigstens zwei pigmentierte Schichten miteinander überlagert auf der Schriftunterlagsschicht aufgebracht werden und diese durch äußerst präzise Tiefensteuerung des Laserstrahls derartig aufgeschlossen werden, daß jeweils die darunterliegende pigmentierte Farbschicht erkennbar ist.

Diese Art der Beschriftung könnte auch auf einem entsprechenden Polymerhintergrund vorgenommen werden. Insgesamt wird mit dem Verfahren nach der Erfindung die bisherige in der Fachwelt geltende Meinung überwunden, ein flächiges klebendes System/Pflaster könne wegen der Empfindlichkeit von Inhaltsstoffen wie Kleber, Zusatzstoffen und Wirkstoffen und der Tiefenwirkung eines Laserstrahls nicht zur Beschriftung mittels Laserstrahl verwendet werden.
Dieses Vorurteil wird mit der Erfindung überholt, weil es damit möglich wird, den Laserstrahl bzw. dessen Intensität so zu steuern, daß er nur die obersten Materialschichten beeinflußt und ansonsten keine Auswirkung auf den weiteren Untergrund hat.

Mit dem erfindungsgemäßen Verfahren werden nunmehr die Bedenken gegen Laserbeschriftung von flächigen klebenden Systemen ausgeräumt, die bisher einer Verwendung auf diesem Sektor im Wege standen. Dabei erweist es sich, daß die Laserbeschriftung zweckmäßig, präzise, ohne Zeitaufwand änderbar und jedem Anwendungsfall anpaßbar ist.

Die Vorteile der Laserbeschriftung nach dem erfindungsgemäßen Verfahren sind:
- mit der berührungsfreien Beschriftung wird die Ausübung von Druck auf ein flächiges klebendes System/Pflaster vorteilhaft vermieden,
- das Verfahren zeichnet sich durch hohe Geschwindigkeit aus,
- die Flexibilität des Beschriftungsverfahrens erlaubt den Austausch und die Anwendung beliebiger Schriftzeichen und Datensätze ohne Zeitverzug,
- die Präzision der Beschriftung erlaubt den Auftrag maschinenlesbarer Markierungen, computergerechter Ziffern, Barcodes oder ähnlicher Zeichen, die der üblichen Vielfalt an Informationen gerecht werden,
- es kann äußerst flexibel in den verschiedenen Produktionsschritten eingesetzt werden,
- es kann besonders rentabel auch auf breiten Klebefolienbahnen eingesetzt werden.

Die Erfindung ist unkompliziert und zweckmäßig und löst in optimaler Weise die eingangs gestellte Aufgabe.

Das Verfahren ist durch eine geeignete Vorrichtung durchführbar, nämlich eine Vorrichtung zum Beschriften eines flächigen klebenden Systems, insbesondere zur Durchführung des Verfahrens nach der Erfindung, umfassend ein Lasergerät im Zusammenwirken mit Mitteln zur Steuerung des Laserstrahls in Richtung und Strahlungsintensität nach Maßgabe einer über einen Datenspeicher und -prozessor verfügenden Führungseinheit, deren Programm
- entweder über Daten aus anderen Produktionsschritten gesteuert wird, oder
- dadurch, daß der Führungseinheit eine Tastatur mit einem Wandler zur unmittelbaren digitalen Eingabe von Schrift- oder anderen Zeichen oder entsprechenden Datensätzen von Hand aufgeschaltet ist, so daß zu jeder Zeit in ein Beschriftungsprogramm eingegriffen werden kann, und manuell beliebige Daten oder Datensätze eingefügt werden können, oder
- bei dem eine Steuerung durch Daten aus anderen Produktionsschritten oder manuell erfolgt.

## Patentansprüche

1. Verfahren zum Beschriften eines flächigen klebenden Systems mit Hilfe eines beweglich geführten Laserstrahls, wobei das System eine zu beschriftende Schicht aus einem Polymer sowie eine darunter liegende Schicht aufweist, die wirkstoffhaltig ist, und die Intensität und Eindringtiefe des Laserstrahls nach Maßgabe der Materialbeschaffenheit des flächigen klebenden Systems so eingestellt werden, daß der Laserstrahl nicht bis zu der wirkstoffhaltigen Schicht des Systems durchdringt und dadurch eine schädliche Beeinflussung der im System enthaltenen Wirkstoffe durch die vom Laserstrahl erzeugte Wärme vermieden wird, **dadurch gekennzeichnet daß**
es sich bei den genannten Wirkstoffen um Wirkstoffe zur systemischen Applikation auf und durch die Haut handelt, und daß das genannte flächige klebende System ein transdermales therapeutisches System ist.

2. Verfahren nach Anspruch 1, **dadurch gekennzeichnet, daß** das System eine gegenüber den Wirkstoffen oder Wasserdampf impermeable Rückschicht aufweist, und daß die Intensität und Eindringtiefe des Laserstrahls so eingestellt werden, daß eine schädliche Perforation der Rückschicht vermieden wird.

3. Verfahren nach Anspruch 1 oder 2, **dadurch gekennzeichnet, daß** die zu beschriftende Materialschicht mit einer Überlagerungsschicht, z.B. einer mit einer aufgedruckten Farbschicht, überdeckt wird, die so ausgewählt ist, daß sie schon bei ver-gleichsweise moderater Laserbestrahlung zerfällt und dabei auf dem Hintergrund der darunterliegenden Materialschicht die gelaserten Schriftzeichen erkennen läßt.

4. Verfahren nach Anspruch 1 oder 2, **dadurch gekennzeichnet, daß** sich unter der zu beschriftenden Materialschicht mindestens eine pigmenthaltige Schicht befindet, bei der jede Schicht so ausgewählt ist, daß sie schon bei vergleichsweise moderater Laserbestrahlung zerfällt und dabei auf dem Hintergrund der darunterliegenden Pigmentschicht die gelaserten Schriftzeichen erkennen läßt.

5. Verfahren nach Anspruch 3, **dadurch gekennzeichnet, daß** auch die Überlagerungsschicht gegenüber der zu beschriftenden Materialschicht mit einer augenfälligen Färbung versehen wird.

6. Verfahren nach einem oder mehreren der Ansprüche 1 bis 5, **dadurch gekennzeichnet, daß** eine Abstimmung der die Wirkungsintensität des Laserstrahls auf der zu beschriftenden Materialschicht bestimmenden Parameter wie Strahlungsenergie, Bündelung und geschwindigkeitsabhängige Einwirkungsdauer des Laserstrahls so vorgenommen wird, daß nur die obersten Materialschichten verändert und ansonsten keine Veränderungen der weiteren Untergrundschichten verursacht werden.

7. Verfahren nach einem oder mehreren der Ansprüche 1 bis 6, **dadurch gekennzeichnet, daß** der Laserstrahl mittels elektromagnetischer Steuerung so geführt wird, daß zu jeder Zeit einzelne Zeichen oder Schriftzüge nach Programm einer zentralen Steuereinheit eingegeben oder geändert, und insbesondere Schriftzeichen oder Datensätze von Hand über eine schreibmaschinenähnliche Tastatur eingegeben werden können.

8. Verfahren nach einem oder mehreren der Ansprüche 1 bis 7, **dadurch gekennzeichnet, daß** der Laserstrahl mittels elektromagnetischer Steuerung so geführt wird, daß dem Programm der Führungseinheit von anderen Produktionsschritten erzeugte Daten transferiert werden.

9. Verfahren nach einem oder mehreren der Ansprüche 1 bis 8, **dadurch gekennzeichnet, daß** zur Erzeugung eines ein- oder mehrfarbigen Schrift- oder Zeichenrnusters wenigstens zwei pigmentierte Schichten miteinander überlagert auf der Schriftunterlagsschicht aufgebracht und diese durch präzise Tiefensteuerung des Laserstrahls derart aufgeschlossen werden, daß jeweils die darunterliegende pigmentierte Farbschicht erkennbar ist.

## Claims

1. Process for inscription of a sheet-like adhesive system with the aid of a movably guided laser beam, said system comprising a layer to be inscribed which is made of a polymer, and a layer which is located thereunder and contains active substance, and the intensity and penetration depth of the laser beam being adjusted according to the material properties of the sheet-like adhesive system in such a way that the laser beam does not penetrate far enough to reach the active substance-containing layer of the system, thereby avoiding a detrimental influence on the active substances contained in the system by the heat generated by the laser beam, **characterised in that** the said active substances are active substances for systemic application to and through the skin, and that the said sheet-like adhesive system is a transdermal therapeutic system.

2. Process according to claim 1, **characterised in that** the system comprises a backing layer impermeable to the active substances or water vapour and that the intensity and the penetration depth of the laser beam are adjusted such that a detrimental perforation of the backing layer is avoided.

3. Process according to Claim 1 or 2, **characterized in that** the material layer to be inscribed is covered with an overlying layer, e.g. a colour layer printed thereon, which is selected such that it disintegrates already at comparatively moderate laser irradiation and in the process visualises the lased characters on the background of the underlying material layer.

4. Process according to Claim 1 or 2, **characterized in that** under the material layer to be inscribed there is at least one pigment-containing layer, each of the layers being selected such that it disintegrates already at comparatively moderate laser irradiation and in the process visualises the lased characters on the background of the underlying pigmented layer.

5. Process according to Claim 3, **characterized in that** the overlying layer too is provided with a conspicuous colour compared to the material layer to be inscribed.

6. Process according to one or more of Claims 1 to 5, **characterized in that** the parameters determining the intensity of the effect of the laser beam on the material layer to be inscribed, such as irradiation energy, concentration and rate-dependent duration of action of the laser beam, are matched in such a way that only the uppermost material layers are modified and apart from that no changes are caused in the further substrate layers.

7. Process according to one or more of Claims 1 to 6, **characterized in that** the laser beam is guided by means of electromagnetic control such that at any time individual signs or groups of characters can be inputted or amended according to a program of a central control unit, and that, in particular, characters or data records can be inputted by hand by means of a keyboard similar to a typewriter.

8. Process according to one or more of Claims 1 to 7, **characterized in that** the laser beam is guided by means of electromagnetic control such that data generated by other production steps are transferred to the programme of the control unit.

9. Process according to one or more of Claims 1 to 8, **characterized in that** to produce a single-coloured or multicoloured pattern of characters or signs, at least two pigmented layers are applied to the inscription substrate layer so as to overly one another, and that these are disintegrated by accurate control of the penetration depth of the laser beam such that the respective underlying pigmented colour layer is visualised.

## Revendications

1. Procédé pour l'inscription d'un système adhésif plat à l'aide d'un faisceau laser guidé en mouvement, le système présentant une couche constituée d'un polymère, qui doit faire l'objet d'une inscription, ainsi qu'une couche sous-jacente qui contient une ou plusieurs substances actives, l'intensité et la profondeur de pénétration du faisceau laser étant réglées, en fonction de la nature matérielle du système adhésif plat, de telle sorte que la pénétration du faisceau laser ne s'étend pas jusqu'à la couche du système contenant une ou plusieurs substances actives afin d'éviter une influence préjudiciable de la chaleur générée par le faisceau laser sur les substances actives que contient le système, **caractérisé en ce qu'**il s'agit, en ce qui concerne les substances actives mentionnées, de substances actives destinées à l'application systémique sur et à travers la peau, et **en ce que** le système adhésif plat mentionné est un système thérapeutique transdermique.

2. Procédé selon la revendication 1, **caractérisé en ce que** le système présente une couche dorsale imperméable aux substances actives ou à la vapeur d'eau, et **en ce que** l'intensité et la profondeur de pénétration du faisceau laser sont réglées de telle sorte que l'on évite une perforation préjudiciable de la couche dorsale.

3. Procédé selon la revendication 1 ou 2, **caractérisé en ce que** la couche de matière qui doit faire l'objet d'une inscription est recouverte d'une couche de revêtement, par exemple d'une couche colorée appliquée par impression qui est sélectionnée de telle sorte qu'elle se décompose déjà en présence d'un rayonnement laser relativement modéré pour ainsi rendre lisibles les inscriptions obtenues par laser sur le substrat de la couche de matière sous-jacente.

4. Procédé selon la revendication 1 ou 2, **caractérisé en ce qu'**au moins une couche pigmentaire est disposée en dessous de la couche de matière qui doit faire l'objet d'une inscription, chaque couche étant sélectionnée de telle sorte qu'elle se décompose déjà sous l'influence d'un rayonnement laser relativement modéré pour ainsi rendre lisibles les inscriptions obtenues par laser sur le substrat de la couche pigmentaire sous-jacente.

5. Procédé selon la revendication 3, **caractérisé en ce que** la couche de revêtement est également munie d'une coloration dont le contraste par rapport à la couche de matière qui doit faire l'objet d'une inscription est visible à l'oeil nu.

6. Procédé selon une ou plusieurs des revendications 1 à 5, **caractérisé en ce qu'**on procède à une adaptation des paramètres déterminant l'intensité d'action du faisceau laser sur la couche de matière qui doit faire l'objet d'une inscription, tels que l'énergie de rayonnement, la focalisation et la durée d'action du faisceau laser en fonction de la vitesse, de telle sorte que seules les couches de matières les plus supérieures sont modifiées et de telle sorte que l'on n'est confronté par ailleurs à aucune modification des autres couches de substrat.

7. Procédé selon une ou plusieurs des revendications 1 à 6, **caractérisé en ce que** le faisceau laser est guidé à l'aide d'une commande électromagnétique de telle sorte que l'on peut entrer ou modifier à tout moment des signes individuels ou des écritures en fonction du programme d'une unité de commande centrale, et en particulier de telle sorte que l'on peut entrer des signes d'écriture ou des jeux de données à la main en passant par un clavier analogue à celui d'une machine à écrire.

8. Procédé selon une ou plusieurs des revendications 1 à 7, **caractérisé en ce que** le faisceau laser est guidé à l'aide d'une commande électromagnétique de telle sorte que l'on peut transférer au programme de l'unité de guidage des données générées à partir d'autres étapes de production.

9. Procédé selon une ou plusieurs des revendications 1 à 8, **caractérisé en ce que**, pour obtenir un modèle d'écriture ou de signes monochrome ou polychrome, on applique au moins deux couches pigmentaires superposées l'une à l'autre sur la couche de substrat d'écriture et on les soumet à une décomposition via un réglage précis de la profondeur du faisceau laser de telle sorte que l'on peut détecter respectivement la couche colorée pigmentaire sous-jacente.
